# EUROPÄISCHE PATENTSCHRIFT

(11) **EP 4 504 167 B1**
(45) Veröffentlichungstag und Bekanntmachung des Hinweises auf die Patenterteilung: **18.02.2026**
(21) Anmeldenummer: 23719002.0
(22) Anmeldetag: 06.04.2023
(51) Int. Cl.: A61K 31/198, A61K 31/718, A61P 17/14, A61K 8/44, A61K 8/73, A61Q 7/00

(54) **ORALE ZUSAMMENSETZUNG ENTHALTEND DIMETHYLGLYCIN ZUR VERBESSERUNG DES ZUSTANDS DER HAARE UND DER KOPFHAUT**
ORAL COMPOSITION CONTAINING DIMETHYLGLYCINE FOR IMPROVING THE CONDITION OF THE HAIR AND SCALP
COMPOSITION ORALE CONTENANT DE LA DIMÉTHYLGLYCINE POUR AMÉLIORER L'ÉTAT DES CHEVEUX ET DU CUIR CHEVELU

(30) Priorität: 06.04.2022 DE 102022108313
(43) Veröffentlichungstag der Anmeldung: 12.02.2025
(73) Patentinhaber: Dr. Kurt Wolff GmbH & Co. KG, 33611 Bielefeld (DE)
(72) Erfinder: SCHULZE ZUR WIESCHE, Erik, 33611 Bielefeld (DE); BECKER, Maike, 33611 Bielefeld (DE); VÖLKER, Jörn Michael, 33611 Bielefeld (DE)
(74) Vertreter: Weickmann & Weickmann PartmbB
(86) Internationale Anmeldenummer: PCT/EP2023/059231
(87) Internationale Veröffentlichungsnummer: WO 2023/194567

(56) Entgegenhaltungen:
- WO-A1-03/026438
- WO-A2-2004/026295
- US-A1- 2007 231 377
- US-A1- 2013 095 125

## Beschreibung

Die vorliegende Erfindung betrifft eine orale Zusammensetzung, die Dimethylglycin und/oder ein Salz von Dimethylglycin und Amylopektin enthält. Zudem betrifft die vorliegende Erfindung die (kosmetische und/oder medizinische) Verwendung dieser Zusammensetzung zur Verbesserung des Zustands der Haare und/oder der Kopfhaut, zur Förderung des Stoffwechsels der Haare und/oder der Kopfhaut, zur Förderung des Haarwachstums und/oder zur Behandlung und/oder Vorbeugung von Haarausfall. Weiterhin betrifft die vorliegende Erfindung ein Nahrungsergänzungsmittel zur Förderung des Stoffwechsels der Haare und/oder der Kopfhaut und/oder zur Förderung des Haarwachstums und/oder zur Behandlung und/oder Vorbeugung von Haarausfall.

Das menschliche Haar hat seine Bedeutung als Schutzfunktion für den Körper mittlerweile zu einem großen Teil verloren. Allerdings hat gesundes Haar weltweit eine große kulturelle Bedeutung für Frauen und Männer und wird häufig als Zeichen von Wohlstand und Gesundheit angesehen. Folglich beeinflusst beispielsweise durch Haarausfall bedingtes schütteres Haar oft die Lebensqualität auf negative Weise.

Dabei sind die Ursachen von Haarausfall vielfältig und reichen von altersbedingtem Haarausfall über genetisch bzw. hormonell bedingte Störungen, Autoimmunstörungen, Stoffwechselstörungen, einschließlich Fehlernährung, bis hin zu Infektionen, Traumen, Tumoren und Medikamenten wie Zytostatika und Antikoagulantien.

Ein durch Hormone wie Androgene hervorgerufener Haarausfall wird als androgenetische Alopezie (Alopecia androgenetica oder auch AGA) bezeichnet und gilt insbesondere bei Männern aber ebenfalls auch bei Frauen als die häufigste Ursache für Haarausfall. Hierbei kann zwischen männlichen und weiblichen Haarausfallmustern unterschieden werden. Andere häufige Formen von nicht vernarbender Alopezie umfassen Telogenes Effluvium (TE), einen diffusen Haarausfall, sowie den akut einsetzenden entzündlich bedingten kreisrunden Haarausfall, der auch als Alopecia areata (AA) bezeichnet wird.

Das Haarwachstum und das Nachwachstum von Haaren hängen von einer ausreichenden Nährstoffversorgung der Haarfollikel ab, welche über das Blut bereitgestellt wird.

Eine verringerte Durchblutung (Mikrozirkulation) der Kopfhaut kann somit Haarausfall begünstigen oder gar verursachen. Bei glatzköpfigen Männern ist beispielsweise die Durchblutung der Vertex -Region der Kopfhaut signifikant geringer als bei Männern mit normaler Kopfhautbehaarung. Diese signifikant verringerte Mikrozirkulation kann also eine Erklärung für den Haarausfall sowie das ausbleibende Nachwachsen der Haare (Übergang von Telogen zu Anagen) beispielsweise bei der androgenetischen Alopezie sein. Insgesamt kann Haarausfall also als eines der ersten klinischen Anzeichen für einen verminderten Blutdurchfluss der peripheren Gefäße angesehen werden.

Andererseits wird auch ein Einfluss von Nährstoffmangel auf die Haarstruktur und das Haarwachstum diskutiert. Zu den Auswirkungen auf das Haarwachstum gehören das akute Telogene Effluvium (TE), eine bekannte Folge von plötzlichem Gewichtsverlust oder verminderter Proteinzufuhr, sowie die diffuse Alopezie, die bei Niacinmangel auftritt. In Studien wurde auch über mögliche Zusammenhänge zwischen Nährstoffmangel und chronischer TE, androgenetischer Alopezie (AGA), weiblichem Haarausfall (FPHL) und Alopecia areata (AA) berichtet (Guo E.L., Katta R. Diet and hair loss: effects of nutrient deficiency and supplement use. Dermatol Pract Concept. 2017;7(1): 1).

Mikronährstoffe wie Vitamine und Mineralstoffe spielen eine wichtige, aber nicht ganz eindeutige Rolle für die normale Entwicklung der Haarfollikel und die Funktion der Immunzellen. Dementsprechend gibt es in zahlreichen Studien Hinweise auf einen Zusammenhang zwischen Haarausfall und Mikronährstoffmangel.

Einen Überblick über die Rolle von Vitaminen und Mineral stoffen wie Vitamin A, Vitamin B, Vitamin C, Vitamin D, Vitamin E, Eisen, Selen und Zink, bei nicht vernarbender Alopezie geben Almohanna et al. (Almohanna, Hind M et al. "The Role of Vitamins and Minerals in Hair Loss: A Review." Dermatology and therapy vol. 9,1 (2019): 51 -70).

Guo und Katta beschreiben die Studienlage im Hinblick auf einen Mangel von bzw. Supplementation mit Eisen, Zink, Niacin, Fettsäuren, Selen, Vitamin D, Vitamin A, Vitamin E, Folsäure, Biotin, Aminosäuren und Proteinen sowie Antioxidantien.

In ähnlicher Weise befasst sich ein Übersichtsartikel von Goldberg und Lenzy mit Veränderungen des Haares aufgrund von allgemeiner Fehlernährung und Mangel an Mikronährstoffen in bestimmten Patientengruppen (Goldberg L.J., Lenzy Y. Nutrition and hair. Clin Dermatol. 2010 Jul -Aug;28(4):412-9).

Eine mögliche Rolle von Nahrungsergänzungsmitteln wie etwa Aminosäuren, Vitaminen und Spurenelementen, essentiellen Fettsäuren, den Coenzymen Q und A, Bioflavonoiden, Inositol, Methyl sulfonylmethan (MSM), Dimethylglycin (DMG), Kieselsäure, Kelp, Grüner Tee-Extrakt, Sägepalme-Extrakt und Weintraubensamenextrakt bei der Vorbeugung von Haarausfall wird schließlich in einem Übersichtsartikel von Jain et al. beschrieben (Jain, Pushpendra & Joshi, Himanshu & Das, Debajyoti. (2012). Drug that Causes Hair Loss and Promotes Hair Growth- A Review. International Journal of Research in Pharmaceutical and Biomedical Sciences. 3. 1476-82).

Ein Mangel an solchen Mikronährstoffen könnte ein veränderbarer Risikofaktor für die Entwicklung, Prävention und Behandlung von Alopezie sein.

Während Nahrungsergänzungsmittel mit Vitaminen und Mineralstoffen relativ preiswert und leicht zugänglich sind, setzt deren Einsatz jedoch voraus zu wissen, welche Vitamine und Mineralien bei der Behandlung von Haarausfall hilfreich sind. Bis zum heutigen Tag sind jedoch nur wenige Studien durchgeführt worden, die die Wirkung einer Mikronährstoffsupplementierung auf das Haarwachstum bei Patienten mit Mikronährstoffmangel und Alopezie untersuchen, um einen Zusammenhang zwischen Haarausfall und Mikronährstoffmangel festzustellen, und es gibt noch viel weniger Literatur über die Auswirkungen von Supplementierung bei Personen ohne Nährstoffmangel.

Zudem gibt es Hinweise, dass eine Überdosierung bestimmter Mikronährstoffe, wie beispielsweise Vitamin A, Vitamin E oder Selen, Haarausfall sogar begünstigen kann (Almohanna et al.; Guo and Katta).

Zusammenfassend können sich Nahrungsergänzungsmittel sowohl positiv als auch negativ auf Haarausfall auswirken, und, sofern kein Nährstoffmangel festgestellt wird, gibt es nur wenig Hinweise darauf, welche Art von Supplementierung geeignet ist, um das Haarwachstum wiederherzustellen oder weiteren Haarausfall zu verhindern.

Daneben gibt es bis heute nur wenige zugelassene pharmazeutische Behandlungsmöglichkeiten für Haarausfall, wie androgenetische Alopezie, und die bereits vorhandenen Behandlungsmöglichkeiten sind entweder nicht ausreichend wirksam oder gehen mit unangenehmen Nebenwirkungen einher. Ähnlich unerfreulich sieht es beispielsweise bei Behandlungsmöglichkeiten für den akut einsetzenden entzündlich bedingten kreisrunden Haarausfall Alopecia areata, und für durch andere Ursachen bedingten Haarausfall aus.

Damit besteht weiterhin ein Bedarf an verbesserten Behandlungsmethoden, insbesondere ein Bedarf an Zusammensetzungen, welche oral gegen Haarausfall eingesetzt werden können, wobei diese Zusammensetzungen keine oder nur vernachlässigbare Nebenwirkungen zeigen sollen. Insbesondere besteht ein Bedarf an Zusammensetzungen in Form von Nahrungsergänzungsmitteln, die leicht erhältlich sind und unkompliziert angewendet werden können. Ebenso besteht ein Bedarf an Zusammensetzungen, welche pharmazeutischer Natur sind und keine Nebenwirkungen zeigen oder welche kosmetischer Natur sind.

WO 03/026438 beschreibt ein Kreatin-Abgabesystem. Das Kreatin-Abgabesystem kann zusätzlich andere bioaktive Inhaltsstoffe wie Nutrazeutika, Kohlenhydrate, Pflanzenstoffe und Vitamine enthalten.

Ausgehend hiervon war es die Aufgabe der vorliegenden Erfindung, eine gut verträgliche Zusammensetzung zur Behandlung von Haaren und der Kopfhaut, insbesondere zur Prävention und/oder Behandlung von Haarausfall, bereitzustellen. Weiterhin sollte sich diese Zusammensetzung oral anwenden lassen und die Nachteile der aus dem Stand der Technik bekannten Zusammensetzungen überwinden.

Diese Aufgabe wurde überraschend durch die Zusammensetzung gemäß Anspruch 1 und deren Verwendung gemäß Anspruch 8 gelöst. Bevorzugte Ausführungsformen gehen aus den abhängigen Ansprüchen hervor.

Erfindungsgemäß wird die Aufgabe durch die Bereitstellung einer oralen Zusammensetzung gelöst, welche
a) Dimethylglycin und/oder ein Salz von Dimethylglycin, und
b) Amylopektin enthält, wobei das Gewichtsverhältnis der Menge von Amylopektin in der Zusammensetzung zur Gesamtmenge von Amylopektin und Amylose in der Zusammensetzung 0,9 bis 1,0 beträgt, wobei die Zusammensetzung 0,1 bis 25,0 Gew.-% Dimethylglycin und/oder ein Salz von Dimethylglycin enthält.

Bei der Komponente b) handelt es sich erfindungsgemäß um das Polysaccharid Amylopektin, welches in Form einer stärkeähnlichen Zusammensetzung bestehend aus Amylopektin und Amylose vorliegen kann, wobei der Anteil von Amylopektin in der stärkeähnlichen Zusammensetzung mindestens 90% beträgt. Der übrige Anteil der stärkeähnlichen Zusammensetzung von bis zu 10% kann Amylose sein.

Erfindungsgemäß beträgt der Anteil von Amylopektin an der Gesamtmenge von Amylopektin und Amylose auch bezogen auf die erfindungsgemäße orale Zusammensetzung mindestens 90%. Entsprechend beträgt der Anteil von Amylose, sofern vorhanden, auch bezogen auf die gesamte erfindungsgemäße orale Zusammensetzung bis zu 10% der Gesamtmenge von Amylopektin und Amylose. Anders ausgedrückt ist gemäß der Definition der Komponente b) erfindungswesentlich, dass die Zusammensetzung einen möglichst hohen Amylopektinanteil im Vergleich zum Amyloseanteil aufweist.

Dementsprechend ergibt sich gemäß der vorliegenden Erfindung ein Gewichtsverhältnis der Menge von Amylopektin in der Zusammensetzung zur Gesamtmenge von Amylopektin und Amylose in der Zusammensetzung von 0,9 bis 1,0, wobei das Gewichtsverhältnis von 1,0 per Definition bedeutet, dass keine Amylose in der Zusammensetzung vorhanden ist.

Es hat sich überraschend gezeigt, dass die erfindungsgemäße Zusammensetzung mit Dimethylglycin und/oder einem Salz von Dimethylglycin sowie einem oder mehreren Amylopektin(en) eine ausgezeichnete Wirksamkeit in der Behandlung von Haarausfall, insbesondere von erblich bedingtem und altersbedingtem Haarausfall, aufweist. Die Zusammensetzung aktiviert die (Kopf)Haut und verbessert die Nährstoff- und Sauerstoffversorgung der (Kopf)Haut und der Haarwurzel deutlich. Zudem ist sie medizinisch und kosmetisch äußerst verträglich, insbesondere ist die Zusammensetzung medizinisch besonders verträglich.

Dimethylglycin (*N,N*-Dimethylglycin) kommt in Pflanzen, Tieren und dem Menschen vor, wobei es im Menschen nur in sehr geringen Mengen gebildet wird. Es entsteht bei einer mehrstufigen Biosynthese von Glycin aus Cholin als Zwischenprodukt durch Transaminierung von Betain mit Betain-Homocystein- Methylase.

N,N-Dimethylglycin, auch (Dimethylamino)essigsäure, wird durch die folgende chemische Formel 1 dargestellt:

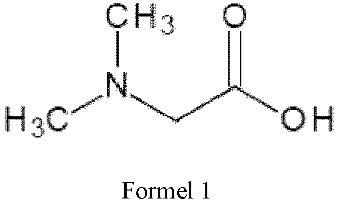

Gemäß der vorliegenden Erfindung wurde überraschenderweise gefunden, dass sich Dimethylglycin und/oder ein Salz von Dimethylglycin nicht nur zur topischen Anwendung eignet, sondern in Kombination mit Amylopektin auch als orales Präparat in hervorragender Weise zur Verbesserung des Zustands der Haare und der Kopfhaut, insbesondere zur Behandlung oder Vorbeugung von Haarausfall geeignet ist.

Dies war insbesondere überraschend, da Stärke, deren Hauptbestandteil Amylopektin ist, im Stand der Technik üblicherweise als inerter Trägerstoff oder Füllstoff und sogar als Placebo in Anwendungsstudien verwendet wurde und Stärke bzw. deren Bestandteilen Amylopektin und Amylose somit eben keine Wirkung zugeschrieben wurde (Morganti, P., et al. (1998). "EFFECT OF GELATIN- CYSTINE AND SERENOA REPENS EXTRACT ON FREE RADICALS LEVEL AND HAIR GROWTH." J Appl Cosmetol 16: 57-64).

Erfindungsgemäß ist nicht nur der Einsatz von Dimethylglycin, sondern auch der von dessen Salzen, Solvaten und Hydraten. Dabei handelt es sich bevorzugt um pharmazeutisch bzw. kosmetisch annehmbare Salze von Dimethylglycin. Das Salz ist besonders bevorzugt ein wasserlösliches Salz mit einer Löslichkeit in Wasser von mindestens 10 g/1 bei 20°C.

In einer bevorzugten Aufführungsform ist das Salz von Dimethylglycin ein Alkali-, Erdalkali- oder Ammoniumsalz von Dimethylglycin.

Beispiele sind Natrium-, Kalium-, Calcium-, Magnesium- und Ammoniumsalze. Bei den Ammoniumsalzen trägt das Ammoniumkation eine bis vier Alkylgruppen mit jeweils unabhängig voneinander 1 bis 4 Kohlenstoffatomen. Bevorzugt sind das Natrium- und Kaliumsalz von Dimethylglycin, insbesondere das Natriumsalz von Dimethylglycin, nämlich Natrium-dimethylglycinat (Natrium-N,N- dimethylglycinat).

In einer alternativ bevorzugten Ausführungsform kann das Salz von Dimethylglycin das Salz einer anorganischen und/oder organischen Säure mit Dimethylglycin sein.

Beispiele für Salze von Dimethylglycin mit einer anorganischen Säure sind das Hydrochlorid, Hydrobromid, Hydroiodid, Hydrogensulfat, Sulfat, Hydrogensulfit, Sulfit, Hydrogencarbonat, Carbonat, Monophosphat, Diphosphat und Triphosphat von Dimethylglycin sowie Mischungen daraus. Insbesondere bevorzugt ist das Hydrochlorid von Dimethylglycin, nämlich Dimethylglycin-hydrochlorid (*N,N*-Dimethylglycinhydrochlorid).

Beispiele für Salze von Dimethylglycin mit einer organischen Säure sind das Acetat, Laktat, Citrat, Succinat, Fumarat, Maleat und Benzoat von Dimethylglycin sowie Mischungen daraus. Erfindungsgemäß bevorzugt ist das Dimethylglycin und/oder Salz von Dimethylglycin ausgewählt aus der Gruppe bestehend aus Dimethylglycin, Natrium- dimethylglycinat und Dimethylglycin-hydrochlorid.

Es wird angenommen, dass Dimethylglycin und/oder ein Salz von Dimethylglycin erfindungsgemäß die Zellaktivität und den Sauerstoffumsatz in den Keratinocyten verbessert und damit auch die Zellaktivität in der (Kopf)Haut und in den Haarfollikeln fördert. Ferner wird die (Kopf)Haut geglättet und die Hautbarriere gestärkt. Es erzielt so erfindungsgemäß eine deutliche haarwurzel- und (kopf)hautstärkende Wirkung, insbesondere bei der Behandlung von alltags- bzw. altersbedingt gestresster oder geschwächter Haut sowie Haarausfall, wie dem erblich bedingten und altersbedingten Haarausfall.

Die erfindungsgemäße Zusammensetzung enthält ferner Amylopektin, wobei das Gewichtsverhältnis der Menge von Amylopektin in der Zusammensetzung zur Gesamtmenge von Amylopektin und Amylose in der Zusammensetzung 0,9 bis 1,0 beträgt. Erfindungsgemäß geeignete Gewichtverhältnisse sind dabei insbesondere: 0,90; 0,91; 0,92; 0,93; 0,94; 0,95; 0,96; 0,97; 0,98; 0,99; 1,00.

In einer bevorzugten Ausführungsform beträgt das Gewichtsverhältnis der Menge von Amylopektin in der Zusammensetzung zur Gesamtmenge von Amylopektin und Amylose in der Zusammensetzung 0,95 bis 1,00, stärker bevorzugt, 0,96 bis 1,00, stärker bevorzugt 0,97 bis 1,00, stärker bevorzugt 0,98 bis 1,00, stärker bevorzugt 0,99 bis 1,00.

Entsprechend beträgt das Gewichtsverhältnis der Menge von Amylose in der Zusammensetzung zur Gesamtmenge von Amylopektin und Amylose in der Zusammensetzung 0 bis 0,1. In einer bevorzugten Ausführungsform beträgt das Gewichtsverhältnis der Menge von Amylose in der Zusammensetzung zur Gesamtmenge von Amylopektin und Amylose in der Zusammensetzung 0 bis 0,05 stärker bevorzugt, 0 bis 0,04, stärker bevorzugt 0 bis 0,03, stärker bevorzugt 0 bis 0,02, stärker bevorzugt 0 bis 0,01.

Der Gehalt von Amylopektin und Amylose kann mittels im Fachgebiet bekannter Methoden bestimmt werden. Entsprechende geeignete Methoden sind beispielsweise in der Publikation Brust H, Orzechowski S, Fettke J. Starch and Glycogen Analyses: Methods and Techniques. Biomolecules. 2020; 10(7): 1020 beschrieben.

Amylopektin ist zusammen mit Amylose Bestandteil der Stärke.

Der Begriff "Stärke" bezeichnet ein Polysaccharid mit der Formel (C₆H₁₀O₂)n, das aus α-D-Glucose-Einheiten besteht. Diese D-Glucose-Einheiten sind über glycosidische Bindungen miteinander verknüpft, wobei über α-l,4-glycosidische Bindungen lineare Ketten gebildet werden, die mittels α-l,6-glycosidischer Bindungen verzweigt sein können. Stärke besteht meist zu 20-30% aus Amylose und zu 70-80% aus Amylopektin.

Stärke ist natürlicher Bestandteil von Kartoffeln, Maniok, Getreide (z.B. Weizen, Mais, Reis) sowie Hülsenfrüchten (z.B. Erbsen, Linsen, weiße oder rote Bohnen, Kichererbsen).

Amylose bezeichnet ein Polysaccharid aus α-D-Glucose-Monomeren, die lineare Ketten mit helikaler Struktur bilden. Amylose hat eine molare Masse zwischen
100.000 und 1.000.000 g/mol, entsprechend etwa 1000 α-D-Glucose-Monomeren (bei Getreidestärken) bis 4500 α-D-Glucose-Monomeren (bei Kartoffelstärken), die α-l,4-glycosidisch miteinander verbunden sind. Bei hohen molaren Massen treten auch vereinzelte α-l,6-glycosidische Verzweigungen auf.

Amylopektin bezeichnet ein Polysaccharid aus α-D-Glucose-Monomeren, die stark verzweigte Strukturen bilden. Die α-D-Glucose-Monomere sind dabei α-1,4- glycosidisch miteinander verbunden. Etwa alle 10-50 Monomere, bevorzugt etwa alle 10-30 Monomere, stärker bevorzugt etwa alle 10-25 Monomere, stärker bevorzugt etwa alle 10-20 Monomere, am meisten bevorzugt etwa alle 12-15 Monomere ist eine Seitenkette α-l,6-glycosidisch verknüpft, wodurch eine baumartige Verzweigung entsteht. Diese Seitenketten können kurz (12 bis 20 Glucose-Einheiten), lang (30 bis 45 Glucose-Einheiten) und sehr lang (durchschnittlich 60 Glucose-Einheiten) sein (F. A. Schüll: Einfluss spezifischer Eigenschaften der Stärke auf den Brauprozess, Dissertation, Technische Universität München 2012, S. 12 f). Aufgrund dieses Unterschiedes in der Struktur bildet Amylopektin auf molekularer Ebene Knäuel- bzw. verzweigte Strukturen aus, die Verzweigungspunkte liegen hauptsächlich am C6-Atom (Habermehl, Hammann, Krebs: Naturstoffchemie. Eine Einführung. 2. Auflage. Springer, Berlin 2002, ISBN 978-3-540-43952-3). Amylopektin weist eine hohe molare Masse von 10.000.000 bis 200.000.000 g/mol auf(C. Bächtle, P. Winkler and B. Stellbrink: Eine Frage der richtigen Stärke. In: Chemie in unserer Zeit 45, 250-255 (2011); F. A. Schüll: Einfluss spezifischer Eigenschaften der Stärke auf den Brauprozess, Dissertation, Technische Universität München 2012, S. 12: 3,0-4, 1 Mio g/mol für Amylopektin aus Gerste).

Der Begriff "hochverzweigtes Amylopektin" bezeichnet ein Polysaccharid aus α-D- Glucose-Monomeren mit besonders stark verzweigten Strukturen. Die α-D-Glucose- Monomere sind dabei a-l,4-glycosidisch miteinander verbunden, etwa alle 10-30 Monomere, bevorzugt etwa alle 10-25 Monomere, stärker bevorzugt etwa alle 10-20 Monomere, am meisten bevorzugt etwa alle 12-15 Monomere ist eine Seitenkette α-l,6-glycosidisch verknüpft, wodurch eine baumartige Verzweigung entsteht.

Wie hierin verwendet, bezeichnen die Begriffe "Amylose" und "Amylopektin" aus α-D-Glucose-Monomeren bestehende Polysaccharide entsprechend den oben genannten Definitionen, deren Bedeutung sich nicht überlappt.

Die Gewinnung von Amylopektin kann durch Trennung von Amylose und Amylopektin aus natürlicher pflanzlicher Stärke mittels chemischer, physikalischer oder enzymatischer Verfahren erfolgen, zum Beispiel durch enzymatische Behandlung mit Amylasen. Die Eigenschaften von Amylopektin können ferner mittels ebensolcher Verfahren modifiziert werden.

Erfindungsgemäß wird Amylopektin aus natürlicher pflanzlicher Stärke ausgewählt aus Kartoffelstärke (Amylum Solani), Weizenstärke (Amylum Tritici), Reisstärke (Amylum Oryzae), Maisstärke (Amylum Maydis), Hirsestärke (Amylum Sorghum) oder Kombinationen hiervon gewonnen. In einer bevorzugten Ausführungsform handelt es sich bei dem in der oralen Zusammensetzung der vorliegenden Erfindung verwendeten Amylopektin um Amylopektin aus Mais-, Kartoffel- oder Hirsestärke.

Erfindungsgemäß ist es ferner möglich, dass es sich bei dem Amylopektin nicht um ein Wachsgetreide handelt bzw. das Amylopektin nicht aus einem Wachsgetreide gewonnen wird. In einer Ausführungsform handelt es sich bei dem Amylopektin nicht um Wachsmais bzw. wird das Amylopektin nicht aus Wachsmais gewonnen.

Gemäß der vorliegenden Erfindung enthält das Amylopektin eine oder mehrere Art(en) von Amylopektin, die sich hinsichtlich des Verzweigungsgrades (Häufigkeit der Verzweigungen sowie Länge der Seitenketten) und des Molekulargewichts unterscheiden können.

In einer bevorzugten Ausführungsform ist das Amylopektin aus mindestens 1000 D- Glucose-Monomeren, bevorzugt mindestens 4500, stärker bevorzugt mindestens 5.000, stärker bevorzugt mindestens 10.000 D -Glucose-Monomeren zusammengesetzt, welche α-l,4-glycosidisch miteinander verbunden sind.

In einer weiteren bevorzugten Ausführungsform enthält das Amylopektin etwa alle 10-50 Monomere, bevorzugt etwa alle 10-30 Monomere, stärker bevorzugt etwa alle 10-25 Monomere, stärker bevorzugt etwa alle 10-20 Monomere, am meisten bevorzugt etwa alle 12-15 Monomere eine Seitenkette aus D-Glucose-Monomeren, die α-l,6-glycosidisch verknüpft ist.

In einer weiteren bevorzugten Ausführungsform umfassen die Seitenketten des Amylopektins jeweils etwa 5-60 Monomere, bevorzugt jeweils etwa 10-30 Monomere, stärker bevorzugt jeweils etwa 10-20 Monomere, stärker bevorzugt jeweils etwa 10-15 Monomere. Bei den genannten Monomeren handelt es sich jeweils um α-D-Glucose-Monomere.

Der Begriff "D-Glucose-Monomere" bezieht sich, wie hierin verwendet, auf α-D- Glucose-Monomere.

In einer weiteren bevorzugten Ausführungsform weist das Amylopektin eine molare Masse von mindestens 100.000 g/mol auf, bevorzugt von mindestens 100.000 g/mol bis zu 200.000.000 g/mol, stärker bevorzugt von mindestens 1.000.000 g/mol bis zu 200.000.000 g/mol, stärker bevorzugt von mindestens 10.000.000 g/mol bis zu 200.000.000 g/mol.

Die erfindungsgemäße Zusammensetzung kann neben dem Amylopektin b) weitere Polysaccharide enthalten, unter der Voraussetzung, dass das Gewichtsverhältnis der Menge von Amylopektin in der Zusammensetzung zur Gesamtmenge von Amylopektin und Amylose in der Zusammensetzung 0,9 bis 1,0 beträgt.

In einer bevorzugten Ausführungsform enthält die erfindungsgemäße Zusammensetzung keine herkömmliche Stärke, wobei der Begriff "herkömmliche Stärke", wie hierin verwendet, Stärke mit einer Zusammensetzung aus 20-30% Amylose und 70-80% Amylopektin bezeichnet.

In einer weiteren bevorzugten Ausführungsform enthält die erfindungsgemäße Zusammensetzung keine Amylose.

Die erfindungsgemäße Zusammensetzung enthält Dimethylglycin und/oder ein Salz von Dimethylglycin in einem Anteil von 0,1 Gew.-% bis 25,0 Gew - %, bezogen auf das Gesamtgewicht der Zusammensetzung. In einer bevorzugten Ausführungsform enthält die erfindungsgemäße Zusammensetzung Dimethylglycin und/oder ein Salz von Dimethylglycin in einem Anteil von 1 Gew.-% bis 20,0 Gew - %, bevorzugter von 2 Gew.-% bis 18,0 Gew. -% jeweils bezogen das Gesamtgewicht der Zusammensetzung. In einer bevorzugten Ausführungsform der Erfindung kann die erfindungsgemäße Zusammensetzung 1 Gew.-%, 2 Gew.-%, 3 Gew.-%, 4 Gew - %, 5 Gew.-%, 6 Gew.-%, 7 Gew.-%, 8 Gew.-%, 9 Gew.-%, 10 Gew.-%, 11 Gew - %, 12 Gew.-%, 13 Gew.-%, 14 Gew.-%, 15 Gew.-%, 20 Gew.-% oder 25 Gew.-% Dimethylglycin und/oder ein Salz von Dimethylglycin enthalten, jeweils bezogen auf das Gesamtgewicht der Zusammensetzung.

Bevorzugt enthält die erfindungsgemäße Zusammensetzung Dimethylglycin und/oder ein Salz von Dimethylglycin als chemischen Reinstoff, einschließlich der jeweiligen Solvate und Hydrate (z.B. des Dihydrats von Natriumdimethyl glycinat), da so die Reinheit der Zusammensetzung erhöht und das Auftreten von unerwünschten Nebenwirkungen vermindert werden kann. Aus diesem Grund enthält die erfindungsgemäße Zusammensetzung bevorzugt die chemischen Derivate von Dimethylglycin, ausgewählt aus Methylglycin, Trimethylglycin, (2-Hydroxyethyl)- trimethyl ammonium, und Trimethyl-hydroxybutyrobetain, in Konzentrationen von weniger als 0,01 Gew.-%, bezogen auf das Gesamtgewicht der Zusammensetzung. Besonders bevorzugt sind die erfindungsgemäßen Zusammensetzungen vollständig frei von diesen Derivaten.

In einer bevorzugten Ausführungsform enthält die erfindungsgemäße Zusammensetzung c) mindestens einen weiteren Wirkstoff, wobei der mindestens eine weitere Wirkstoff ausgewählt ist aus Amla-Extrakt, Amylodextrin, β- Carotin, Biotin, Carnitin, Curcumin, Echinacea, Ectoin, Eisen, Folsäure, Granatapfel -Extrakt, Grüntee-Extrakt, Hirseextrakt, Hirseöl, Kürbiskernöl, Kreatin, L-Cystein, L-Lysin, Niacin, Niacinamid, Pantolacton, Panthothensäure, Piperin, Procyanidin, Rosmarin-Extrakt, Sägepalme -Extrakt, Schachtelhalm-Extrakt, Selen, Silicium, Taurin, Tocopherylacetat, Tomatenextrakt, Ubichinon-10, Vitamin Bl, Vitamin B2, Vitamin B6, Vitamin B12, Vitamin C, Vitamin E, Zink und Kombinationen hiervon, insbesondere ausgewählt aus Zink, Biotin, Hirseextrakt, Selen und Kombinationen hiervon.

Amla-Extrakt ist ein Extrakt aus der Indischen Stachelbeere (Phyllanthus emblica). In der erfindungsgemäßen Zusammensetzung kann Amla-Extrakt in einer Menge von 0,1-50,0 Gew.-%, bevorzugt 1-30 Gew.-%, stärker bevorzugt 5-25 Gew.-% enthalten sein, jeweils bezogen auf das Gesamtgewicht der Zusammensetzung.

In der erfindungsgemäßen Zusammensetzung ist Amylose bevorzugt in einer Menge von 0-5 Gew.-% enthalten, bezogen auf das Gesamtgewicht der Zusammensetzung. Amylodextrin ist ein Umwandlungsprodukt der Stärke mittels verdünnter Säuren. Das Amylodextrin (6 C₆H₁₀O₆ + 2 H₂O) ist eine der Stärke isomere Verbindung. Amylodextrin kann auch als Endprodukt der Hydrolyse von Amylopektin durch β- Amylase gewonnen werden. In der erfindungsgemäßen Zusammensetzung können ein oder mehrere Amylodextrin(e) in einer Menge von 0,1-10,0 Gew.-%, bevorzugt 1- 5 Gew.-% enthalten sein, jeweils bezogen auf das Gesamtgewicht der Zusammensetzung.

β-Carotin ist eine Vorstufe von Vitamin A und wird deshalb auch als Provitamin A bezeichnet. In der erfindungsgemäßen Zusammensetzung kann β-Carotin in einer Menge von 0,01-1,0 Gew. -%, bevorzugt 0,05-0,5 Gew.-% enthalten sein, jeweils bezogen auf das Gesamtgewicht der Zusammensetzung.

Biotin, welches auch als Vitamin B₇ oder Vitamin H bezeichnet wird, ist ein wasserlösliches Vitamin aus dem B-Komplex. Biotin kann erfindungsgemäß Haarausfall weiter vermindern und die Haut stärken. In der erfindungsgemäßen Zusammensetzung kann Biotin in einer Menge von 0,001-10,0 Gew.-%, bevorzugt 0,005-1,0 Gew.-% enthalten sein, jeweils bezogen auf das Gesamtgewicht der Zusammensetzung.

In der erfindungsgemäßen Zusammensetzung kann Carnitin in einer Menge von 0,001 Gew.-% bis 10,0 Gew.-%, bevorzugt 0,005 Gew.-% bis 7,50 Gew.-% enthalten sein, jeweils bezogen auf das Gesamtgewicht der Zusammensetzung.

In der erfindungsgemäßen Zusammensetzung kann Curcumin in einer Menge von 0, 1-20,0 Gew.-%, bevorzugt 1-15 Gew.-% enthalten sein, jeweils bezogen auf das Gesamtgewicht der Zusammensetzung.

Echinacea kann die Blutzirkulation der Kopfhaut anregen und so die Haarfollikel mit Sauerstoff- und nährstoffreichem Blut versorgen, was sich weiter stabilisierend auf die Haare und insbesondere die Haarwurzel auswirkt. In der erfindungsgemäßen Zusammensetzung kann Echinacea in einer Menge von 0,001 Gew.-% bis 10,0 Gew.-%, bevorzugt 0,005 Gew.-% bis 7,50 Gew.-% enthalten sein, jeweils bezogen auf das Gesamtgewicht der Zusammensetzung.

Ectoin ist eine zyklische Aminosäure und stabilisiert erfindungsgemäß die natürliche Struktur von Haaren weiter. In der erfindungsgemäßen Zusammensetzung kann Ectoin in einer Menge von 0,001 Gew.-% bis 10,0 Gew.-%, bevorzugt 0,005 Gew.-% bis 7,50 Gew.-% enthalten sein, jeweils bezogen auf das Gesamtgewicht der Zusammensetzung.

In der erfindungsgemäßen Zusammensetzung kann Eisen in einer Menge von 0,01- l,0 Gew.-%, bevorzugt 0,05-0,5 Gew.-% enthalten sein, jeweils bezogen auf das Gesamtgewicht der Zusammensetzung.

In der erfindungsgemäßen Zusammensetzung kann Folsäure in einer Menge von 0,001-1,0 Gew.-%, bevorzugt 0,05-0,5 Gew.-% enthalten sein, jeweils bezogen auf das Gesamtgewicht der Zusammensetzung.

In der erfindungsgemäßen Zusammensetzung kann Granatapfel -Extrakt in einer Menge von 0,1-20,0 Gew. -%, bevorzugt 1-15 Gew.-% enthalten sein, jeweils bezogen auf das Gesamtgewicht der Zusammensetzung.

In der erfindungsgemäßen Zusammensetzung kann Grüner Tee-Extrakt in einer Menge von 0,1-10,0 Gew.-%, bevorzugt 1, 0-5,0 Gew.-%, jeweils bezogen auf das Gesamtgewicht der Zusammensetzung.

In der erfindungsgemäßen Zusammensetzung kann Hirseextrakt in einer Menge von 0,01-40,0 Gew. -%, bevorzugt 1-35 Gew. -%, stärker bevorzugt 5-30 Gew. -% enthalten sein, jeweils bezogen auf das Gesamtgewicht der Zusammensetzung.

In der erfindungsgemäßen Zusammensetzung kann Hirseöl in einer Menge von 0,01- 30,0 Gew.-%, bevorzugt 1-15 Gew.-% enthalten sein, jeweils bezogen auf das Gesamtgewicht der Zusammensetzung.

In der erfindungsgemäßen Zusammensetzung kann Kürbiskernöl in einer Menge von 0,1-20,0 Gew.-%, bevorzugt 1-15 Gew.-% enthalten sein, jeweils bezogen auf das Gesamtgewicht der Zusammensetzung.

In der erfindungsgemäßen Zusammensetzung kann Kreatin 0,001 Gew.-% bis 10,0 Gew.-%, bevorzugt 0,005 Gew.-% bis 7,50 Gew.-% enthalten sein, jeweils bezogen auf das Gesamtgewicht der Zusammensetzung.

In der erfindungsgemäßen Zusammensetzung kann L-Cystein in einer Menge von 0,01-20,0 Gew.-%, bevorzugt 0, 1-1,0 Gew.-% enthalten sein, jeweils bezogen auf das Gesamtgewicht der Zusammensetzung.

In der erfindungsgemäßen Zusammensetzung kann L-Lysin in einer Menge von 0,1- 20,0 Gew.-%, bevorzugt 1-15 Gew.-% enthalten sein, jeweils bezogen auf das Gesamtgewicht der Zusammensetzung.

In der erfindungsgemäßen Zusammensetzung kann Niacin in einer Menge von 0, 1- 10,0 Gew.-%, bevorzugt 0, 5-5,0 Gew.-% enthalten sein, jeweils bezogen auf das Gesamtgewicht der Zusammensetzung.

Niacinamid (auch Nicotinamid) ist das Amid der Nicotinsäure und wird auch als Vitamin B3 bezeichnet. Neben anderen Eigenschaften wie beispielweise einer Verringerung von oxidativem Stress hat das Niacinamid erfindungsgemäß eine das Haarwachstum stimulierende Wirkung. In der erfindungsgemäßen Zusammensetzung kann Niacinamid 0,001 Gew.-% bis 10,0 Gew.-%, bevorzugt 0,005 Gew.-% bis 7,50 Gew.-% enthalten sein, jeweils bezogen auf das Gesamtgewicht der Zusammensetzung.

Pantolacton entstammt der Gruppe der substituierten Lactone und regt erfindungsgemäß die Wachstumsfaktoren der Haarwurzeln weiter an. In der erfindungsgemäßen Zusammensetzung kann Pantolacton 0,001 Gew.-% bis 10,0 Gew.-%, bevorzugt 0,005 Gew.-% bis 7,50 Gew.-% enthalten sein, jeweils bezogen auf das Gesamtgewicht der Zusammensetzung.

In der erfindungsgemäßen Zusammensetzung kann Panthothensäure in einer Menge von 0,01-10,0 Gew.-%, bevorzugt 0, 1 -2,0 Gew.-% enthalten sein, jeweils bezogen auf das Gesamtgewicht der Zusammensetzung.

In der erfindungsgemäßen Zusammensetzung kann Piperin in einer Menge von 0,01- 5,0 Gew.-%, bevorzugt 0,05-2,0 Gew.-% enthalten sein, jeweils bezogen auf das Gesamtgewicht der Zusammensetzung.

In der erfindungsgemäßen Zusammensetzung kann Procyanidin in einer Menge von 0, 1-20,0 Gew.-%, bevorzugt 1-15 Gew.-% enthalten sein, jeweils bezogen auf das Gesamtgewicht der Zusammensetzung.

In der erfindungsgemäßen Zusammensetzung kann Rosmarin-Extrakt in einer Menge von 0,001 -20,0 Gew.-%, bevorzugt 0,01-5,0 Gew.-% enthalten sein, jeweils bezogen auf das Gesamtgewicht der Zusammensetzung.

In der erfindungsgemäßen Zusammensetzung kann Sägepalme-Extrakt in einer Menge von 0,1-20,0 Gew. -%, bevorzugt 1-15 Gew.-% enthalten sein, jeweils bezogen auf das Gesamtgewicht der Zusammensetzung.

In der erfindungsgemäßen Zusammensetzung kann Schachtelhalm-Extrakt in einer Menge von 0,01-1,0 Gew.-%, bevorzugt 0, 1-1,0 Gew.-% enthalten sein, jeweils bezogen auf das Gesamtgewicht der Zusammensetzung.

In der erfindungsgemäßen Zusammensetzung kann Selen in einer Menge von 0,0001-0,5 Gew.-%, bevorzugt 0,001-0,01 Gew.-% enthalten sein, jeweils bezogen auf das Gesamtgewicht der Zusammensetzung.

In der erfindungsgemäßen Zusammensetzung kann Silicium (Kieselsäuren) in einer Menge von 0,01-10,0 Gew. -%, bevorzugt 0,1-5,0 Gew.-% enthalten sein, jeweils bezogen auf das Gesamtgewicht der Zusammensetzung.

Taurin oder 2-Aminoethansulfonsäure wirkt erfindungsgemäß als Antioxidans ebenfalls weiter stabilisierend auf die Haut und Haare und insbesondere die Haarwurzel. In der erfindungsgemäßen Zusammensetzung kann Taurin 0,001 Gew. -% bis 10,0 Gew.-%, bevorzugt 0,005 Gew.-% bis 7,50 Gew.-% enthalten sein, jeweils bezogen auf das Gesamtgewicht der Zusammensetzung.

Tocopherylacetat zeigt antioxidative Eigenschaften und wirkt erfindungsgemäß weiter stabilisierend auf die Haut und die Haare und insbesondere die Haarwurzel. In der erfindungsgemäßen Zusammensetzung kann Tocopherylacetat 0,001 Gew.-% bis 10,0 Gew.-%, bevorzugt 0,005 Gew.-% bis 7,50 Gew.-% enthalten sein, jeweils bezogen auf das Gesamtgewicht der Zusammensetzung.

In der erfindungsgemäßen Zusammensetzung kann Tomatenextrakt in einer Menge von 0,1-20,0 Gew.-%, bevorzugt 1-15 Gew.-% enthalten sein, jeweils bezogen auf das Gesamtgewicht der Zusammensetzung.

Ubichinon-10 (Q10 oder Coenzym Q10) ist ein Chinon-Derivat. Das zum Ubichinon-Pool gehörende Q10 gilt als Antioxidans und wirkt erfindungsgemäß stabilisierend auf die Haut und Haare und insbesondere die Haarwurzel. In der erfindungsgemäßen Zusammensetzung kann Ubichinon-10 0,001 Gew.-% bis 10,0 Gew.-%, bevorzugt 0,005 Gew.-% bis 7,50 Gew.-% enthalten sein, jeweils bezogen auf das Gesamtgewicht der Zusammensetzung.

In der erfindungsgemäßen Zusammensetzung kann Vitamin Bl (Thiamin) in einer Menge von 0,01-5,0 Gew. -%, bevorzugt 0,05-1,0 Gew.-% enthalten sein, jeweils bezogen auf das Gesamtgewicht der Zusammensetzung.

In der erfindungsgemäßen Zusammensetzung kann Vitamin B2 (Riboflavin) in einer Menge von 0,01-5,0 Gew. -%, bevorzugt 0,05-1,0 Gew.-% enthalten sein, jeweils bezogen auf das Gesamtgewicht der Zusammensetzung.

In der erfindungsgemäßen Zusammensetzung kann Vitamin B6 in einer Menge von 0,01-5,0 Gew.-%, bevorzugt 0,05-0,5 Gew.-% enthalten sein, jeweils bezogen auf das Gesamtgewicht der Zusammensetzung.

In der erfindungsgemäßen Zusammensetzung kann Vitamin B 12 in einer Menge von 0,00001-0,01 Gew.-%, bevorzugt 0,00005-0,0005 Gew.-% enthalten sein, jeweils bezogen auf das Gesamtgewicht der Zusammensetzung.

In der erfindungsgemäßen Zusammensetzung kann Vitamin C in einer Menge von 0,1-20,0 Gew.-%, bevorzugt 1-15 Gew.-% enthalten sein, jeweils bezogen auf das Gesamtgewicht der Zusammensetzung.

In der erfindungsgemäßen Zusammensetzung kann Vitamin E in einer Menge von 0,01-10,0 Gew.-%, bevorzugt 0, 1-5,0 Gew.-% enthalten sein, jeweils bezogen auf das Gesamtgewicht der Zusammensetzung.

Zink kann als Substanz mit antimikrobieller Wirkung der erfindungsgemäßen Zusammensetzung in einer Menge von 0,01-30,0 Gew.-%, bevorzugt 0, 1-5,0 Gew.-% zugesetzt werden, jeweils bezogen auf das Gesamtgewicht der Zusammensetzung.

Erfindungsgemäß wird die Zusammensetzung oral angewendet. Unter einer oralen Anwendung ist eine innerliche Anwendung zu verstehen.

In einer bevorzugten Ausführungsform ist die Zusammensetzung gemäß der vorliegenden Erfindung ein Nahrungsergänzungsmittel, insbesondere ein Nahrungsergänzungsmittel zur Förderung des Stoffwechsels der Haare und/oder der Kopfhaut und/oder zur Förderung des Haarwachstums und/oder zur Behandlung und/oder Vorbeugung von Haarausfall.

Erfindungsgemäße Zusammensetzungen, insbesondere für Nahrungsergänzungsmittel, sind bevorzugt in Form von Kapseln, Lutschtabletten, Tabletten, Pillen und anderen ähnlichen Darreichungsformen, Pulverbeuteln, Flüssigampullen, Flaschen mit Tropfeinsatz und ähnlichen Darreichungsformen von Flüssigkeiten und Pulvern zur Aufnahme des Produktes.

In einer bevorzugten Ausführungsform sind die Bestandteile, insbesondere a) Dimethylglycin und/oder ein Salz von Dimethylglycin, b) Amylopektin, wobei das Gewichtsverhältnis der Menge von Amylopektin in der Zusammensetzung zur Gesamtmenge von Amylopektin und Amylose in der Zusammensetzung 0,9 bis 1,0 beträgt, wobei die Zusammensetzung 0,1 bis 25,0 Gew.-% Dimethylglycin und/oder ein Salz von Dimethylglycin enthält, und c) der oder die andere(n) Wirkstoff(e) zur Behandlung der Haare und/oder der Kopfhaut, sofern vorhanden, sowie ein oder mehrere geeignete Trägerstoff(e) in der erfindungsgemäßen Zusammensetzung als Tablette verpresst oder in einer Kapsel, z.B. einer Hartgelatinekapsel oder Weichgelatinekapsel, enthalten.

Geeignete Trägerstoffe umfassen, ohne darauf beschränkt zu sein, Pflanzenöle, wie beispielsweise Kokusnussöl, Sojaöl, Sojalecithin, Sonnenblumenöl, Olivenöl, Palmöl, Rapsöl und Leinöl; Bienenwachs; Zuckeralkohole, wie beispielsweise Sorbit, Mannit, Isomalt, Maltit, Lactit, Xylit und Erythrit; Monosaccharide wie Dextrose (Glucose), Mannose, Fructose; Disaccharide wie Maltose; und unverdauliche Kohlenhydrate wie beispielsweise Cellulose und Pektine.

Erfindungsgemäß bevorzugt werden ein oder mehrere Pflanzenöl(e) als Trägerstoff in Kapseln eingesetzt. Für Tabletten kommen vorzugsweise ein oder mehrere Zucker oder Zuckeralkohole, vorzugsweise Xylit zum Einsatz.

Die erfindungsgemäße Zusammensetzung kann durch dem Fachmann bekannte Verfahren hergestellt werden.

Die vorliegende Erfindung bezieht sich weiterhin auf die Verwendung der erfindungsgemäßen Zusammensetzung zur Verbesserung des Zustands der Haare und/oder der Kopfhaut und/oder zur Förderung des Stoffwechsels der Haare und/oder der Kopfhaut und/oder zur Förderung des Haarwachstums und/oder zur Behandlung und/oder Vorbeugung von Haarausfall.

Dabei umfasst der Begriff Haarausfall Alopecia areata (kreisrunden Haarausfall), Alopecia androgenetica (erblich bedingten Haarausfall) bei Frauen und Männern, diffuse Alopezie (diffusen Haarausfall), altersbedingten Haarausfall (senescent Alopecia) und durch Chemotherapie-bedingten Haarausfall.

Besonders bevorzugt ist der zu behandelnde Haarausfall erblich bedingter Haarausfall (Alopecia androgenetica).

Ebenso besonders bevorzugt ist der zu behandelnde Haarausfall altersbedingter Haarausfall (senescent Alopecia).

Die Verwendung ist insbesondere eine kosmetische oder medizinische Verwendung.

Die erfindungsgemäße Verwendung führte dabei überraschenderweise nicht nur zu einer Verringerung des täglichen Haarausfalls, sondern darüber hinaus auch zu einer Zunahme der Haardichte und zu positiven Effekten auf die Haarwurzelaktivität im Vergleich zu einer Kontrollgruppe, wobei die Haardichte sowie die Haarwurzelaktivität durch etablierte Standardverfahren aus dem Bereich Dermatologie vor und nach der Einnahme bestimmt wurden (siehe u.a. Serrano- Falcón C, Fernandez -Pugnaire MA, Serrano-Ortega S. Hair and scalp evaluation: the trichogram.).

Anhand der nachfolgenden Zusammensetzungen soll die Erfindung verdeutlicht werden, ohne sie jedoch auf die speziellen Beispiele einschränken zu wollen.

### Experimenteller Teil

Die folgenden Zusammensetzungen wurden durch für den Fachmann bekannte Maßnahmen hergestellt. Die Menge der Komponenten wurde jeweils so gewählt, dass ihr Gewichtsanteil in der Vormischung den angegebenen Gewichtsanteilen entspricht.

### Beispiel 1

Zusammensetzung in Form einer Tablette, welche die folgenden Komponenten enthält:

| Nährstoff | Einzeldosis | Gew.-% |
|---|---|---|
| Vitamin C | 100 mg | 7,28 |
| Vitamin E (α-Tocopherol) | 6,8 mg | 0,50 |
| Vitamin B2 | 1,4 mg | 0,10 |
| Vitamin B12 | 2 µg | 0,0001 |
| Zink | 20 mg | 1,46 |
| Piperin | 10 mg | 0,73 |
| Panthothensäure | 9 mg | 0,66 |
| Biotin/Vitamin H | 150 µg | 0,01 |
| Folsäure | 312 µg | 0,02 |
| Silicium | 20 mg | 1,46 |
| L-Cystein | 6 mg | 0,44 |
| Hirseextrakt | 400 mg | 29,12 |
| L-Lysin | 100 mg | 7,28 |
| Na-*N,N*-Dimethylglycinat | 200 mg | 14,56 |
| hochverzweigtes Amylopektin | 500 mg | 36,40 |
| Zuckeralkohol als Trägerstoff | q.s. | q.s |

### Beispiel 2

Zusammensetzung in Form einer Kapsel, welche die folgenden Komponenten enthält:

| Nährstoff | Einzeldosis | Gew.-% |
|---|---|---|
| Vitamin C | 110 mg | 11,20 |
| Vitamin E (α-Tocopherol) | 20 mg | 2,04 |
| Niacin | 18 mg | 1,83 |
| Zink | 5 mg | 0,51 |
| Pantothensäure | 6 mg | 0,61 |
| Natürlicher Tomatenextrakt | 66 mg | 6,72 |
| β-Carotin/Provitamin A | 2 mg | 0,20 |
| Vitamin B6 | 1,8 mg | 0,18 |
| Vitamin B2 (Riboflavin) | 1,4 mg | 0,14 |
| Vitamin B1 (Thiamin) | 1,3 mg | 0,13 |
| Folsäure | 312 µg | 0,03 |
| Biotin | 150 µg | 0,02 |
| Selen | 32 µg | 0,0033 |
| Vitamin B12 | 2 µg | 0,0002 |
| *N,N*-Dimethylglycin - hydrochlorid | 150 mg | 15,28 |
| hochverzweigtes Amylopektin | 600 mg | 61,10 |
| Pflanzenöl als Trägerstoff | q.s | q.s |

### Beispiel 3

Zusammensetzung in Form einer Kapsel, welche die folgenden Komponenten enthält:

| Nährstoff | Einzeldosis | Gew.-% |
|---|---|---|
| Biotin | 1 | 0,02 |
| Vitamin C | 100 | 1,98 |
| Vitamin E (α-Tocopherol) | 7 | 0,14 |
| Zink | 20 | 0,40 |
| Selen | 0,2 | 0,004 |
| Eisen | 8 | 0,16 |
| Silicium | 20 | 0,40 |
| Sägepalme-Extrakt | 500 | 9,89 |
| Schachtelhalm-Extrakt | 25 | 0,49 |
| Amla Extrakt | 1000 | 19,79 |
| Kürbiskernöl | 400 | 7,91 |
| Procyanidin | 400 | 7,91 |
| Grüner Tee Extrakt | 200 | 3,96 |
| Rosmarinextrakt | 3 | 0,06 |
| Curcumin | 500 | 9,89 |
| Piperin | 10 | 0,20 |
| Granatapfelextrakt | 500 | 9,89 |
| Hirseöl | 400 | 7,91 |
| L-Cystein | 10 | 0,20 |
| L-Lysin | 200 | 3,96 |
| *N,N*-Dimethylglycin - hydrochlorid | 150 | 2,97 |
| hochverzweigtes Amylopektin | 600 | 11,87 |
| Pflanzenöl als Trägerstoff | q.s | q.s |

### Referenzbeispiel 1

Zusammensetzung in Form einer Tablette (ohne Na-Dimethylglycinat und hochverzweigtes Amylopektin), welche die folgenden Komponenten enthält:

| Nährstoff | Einzeldosis | Gew.-% |
|---|---|---|
| Vitamin C | 100 mg | 7,28 |
| Vitamin E (α-Tocopherol) | 6,8 mg | 0,5 0 |
| Vitamin B2 | 1,4 mg | 0,10 |
| Vitamin B12 | 2 µg | 0,0001 |
| Zink | 20 mg | 1,46 |
| Piperin | 10 mg | 0,73 |
| Panthothensäure | 9 mg | 0,66 |
| Biotin/Vitamin H | 150 µg | 0,01 |
| Folsäure | 312 µg | 0,02 |
| Silicium | 20 mg | 1,46 |
| L-Cystein | 6 mg | 0,44 |
| Hirseextrakt | 400 mg | 29,12 |
| Lysin | 100 mg | 7,28 |
| Zuckeralkohol als Trägerstoff | q.s | q.s |

### Studiendesign und -ergebnisse

1. Im Rahmen einer in vitro Untersuchung wurde die Wirkung von Na- Dimethylglycinat in Kombination mit hochverzweigtem Amylopektin im Zellkulturmodell mit humanen hornbildenden Keratinozyten-Zellen untersucht. Hierfür wurden HaCaT -Zellen für 1, 3, 5, und 7 Tage in DMEM-Medium (inklusive fötalem Kälberserum und einem Antibiotika-Antimykotika-Mix) kultiviert und u.a. die Viabilität und Proliferation der Zellen durch geeignete Messmethoden bestimmt sowie die Expression der für das Zellwachstum relevanten Wachstumsfaktoren bestimmt.

### Nachweis der Viabilität:

Für diese Messung wurde ein sogenannter MTT-Assay verwendet, um die zelluläre Stoffwechselaktivität als Indikator für die Lebensfähigkeit und Zytotoxizität der Zellen zu bestimmen. Dieser kolorimetrische Assay basiert auf der Reduktion eines gelben Tetrazoliumsalzes (3-(4,5-Dimethylthiazol-2-yl)-2,5-diphenyltetrazoliumbromid oder MTT) zu violetten Formazan-Kristallen durch metabolisch aktive Zellen.

Humane epidermale Keratinozyten-Zellen (HaCaT) wurden in einer 96-Well Platte mit einer Zelldichte von 5.000 Zellen/Well ausgesät und in Medium (DMEM mit 10% FBS, 1% Penicillin-Streptomycin, 0.5% Fungizone) kultiviert. Am nächsten Tag (= Startzeitpunkt, Behandlungsbeginn) sowie nach weiteren 24 h sowie 48 h und 72 h Kultivierung bei 37°C und 5 Vol.-% CO2 wurde das Zellkulturmedium ohne (Kontrolle) bzw. mit den jeweils bereits enthaltenen Wirkstoff-Konzentrationen von Natrium - Dimethylglycinat (DMG), hochverzweigtem Amylopektin und der Kombination aus DMG und hochverzweigtem Amylopektin gewechselt. Die Messung der Viabilität erfolgte analog zu bereits publizierten Untersuchungen in B. I. Tóth, N. Dobrosi, A. Dajnoki, G. Czifra, A. Oläh, A. G. Szöllösi, I. Juhäsz, K. Sugawara, R. Paus, T. Biro, J Invest Dermatol 2011, 131, 1095-1104.

### Nachweis der Proliferation:

Für die Messung der Proliferation wurde ein sogenannter CyQUANT- Assay durchgeführt. Bei diesem fluoreszenz-basierten Assay bindet der verwendete Fluoreszenzfarbstoff an DNA (engl. deoxyribonucleic acid), wobei der Gehalt an zellulärer DNA ein direktes Maß für die Anzahl an Zellen innerhalb einer Probe ist.

Humane epidermale Keratinozyten-Zellen (HaCaT) wurden in einer 96-Well Platte mit einer Zelldichte von 5.000 Zellen/Well ausgesät und in Medium (DMEM mit 10% FBS, 1% Penicillin-Streptomycin, 0.5% Fungizone) kultiviert. Am nächsten Tag (= Startzeitpunkt, Behandlungsbeginn) sowie nach weiteren 24 h sowie 48 h und 72 h Kultivierung bei 37°C und 5 Vol.-% CO2 wurde das Zellkulturmedium ohne (Kontrolle) bzw. mit den jeweils bereits enthaltenen Wirkstoff-Konzentrationen von Natrium - Dimethylglycinat (DMG), hochverzweigtem Amylopektin und der Kombination aus DMG und hochverzweigtem Amylopektin gewechselt. Die Messung der Proliferation erfolgte analog zu bereits publizierten Untersuchungen in A. Oläh, B. I. Tóth, I. Borbirö, K. Sugawara, A. G. Szöllösi, G. Czifra, B. Pal, L. Ambrus, J. Kloepper, E. Camera, The Journal of clinical investigation 2014, 124, 3713-3724.

### Nachweis der Genexpression:

Humane epidermale Keratinozyten-Zellen (HaCaT) wurden in einer 6-Well Platte mit einer Zelldichte von 140.000 Zellen/Well ausgesät und in Medium (DMEM mit 10% FBS, 1% Penicillin-Streptomycin, 0.5% Fungizone) bei 37°C und 5 Vol.-% CO2 kultiviert. Am nächsten Tag (= Startzeitpunkt, Behandlungsbeginn) wurde das Zellkulturmedium ohne (Kontrolle) bzw. mit den jeweils bereits enthaltenen Wirkstoff- Konzentrationen von Natrium-Dimethylglycinat (DMG), hochverzweigtem Amylopektin und der Kombination aus DMG und hochverzweigtem Amylopektin gewechselt und die Zellen nach 24 h geerntet. Die Messung der Genexpression erfolgte mittels qRT-PCR basierend auf bereits publizierten Untersuchungen in B. V. Diaz, M.-C. Lenoir, A. Ladoux, C. Frelin, M. Démarchez, S. Michel, Journal of Biological Chemistry 2000, 275, 642-650.

Es hat sich gezeigt, dass mit Na-Dimethylglycinat in Kombination mit hochverzweigtem Amylopektin die für das Wachstum relevanten Parameter der HaCaT -Zellen positiv beeinflußt wurden und die Expression der Wachstumsfaktoren (beispielsweise HGF, KGF, VEGF, GM-CSF) signifikant gesteigert wurde gegenüber der Behandlung von HaCaT-Zellen mit nur Na-Dimethylglycinat oder mit nur hochverzweigtem Amylopektin.

2. Die erfindungsgemäße Zusammensetzung aus Beispiel 1 (Verum - Zusammensetzung mit Natrium -Dimethylglycinat und hochverzweigtem Amylopektin) und die Zusammensetzung aus Referenzbeispiel 1 (Placebo - Zusammensetzung ohne Natrium-Dimethylglycinat und hochverzweigtes Amylopektin) wurden zudem im Rahmen einer randomisierten, kontrollierten und verbündeten Anwendungs Studie an einem Probandenkollektiv von insgesamt 60 Männern und Frauen für 6 Monate eingenommen. Die Probanden wurden zur subjektiven Bewertung des Rückgangs ihres Haarausfalls nach Beendigung der Einnahme befragt, wobei diese Ergebnisse mit Hilfe von Fragebögen festgehalten wurden. Gleichzeitig wurde die Haardichte sowie die Haarwurzelaktivität durch etablierte Standardverfahren aus dem Bereich Dermatologie vor und nach der Einnahme bestimmt (siehe u.a. Serrano-Falcón C, Fernández -Pugnaire MA, Serrano-Ortega S. Hair and scalp evaluation: the trichogram.). Während bzw. nach der oralen Einnahme der erfindungsgemäßen Zusammensetzung aus Beispiel 1 gaben mehr Probanden und Probandinnen an, dass weniger Haare ausgefallen sind im Vergleich zur Kontrollgruppe unter oraler Einnahme der Placebo- Zusammensetzung. Im Rahmen der objektiven Messungen zeigte sich, dass unter oraler Einnahme der erfindungsgemäßen Zusammensetzung aus Beispiel 1 eine Zunahme der Haardichte und positive Effekte auf Haarwurzelaktivität nachgewiesen wurden im Vergleich zur Kontrollgruppe.

## Patentansprüche

1. Orale Zusammensetzung enthaltend
a) Dimethylglycin und/oder ein Salz von Dimethylglycin, und
b) Amylopektin, wobei das Gewichtsverhältnis der Menge von Amylopektin in der Zusammensetzung zur Gesamtmenge von Amylopektin und Amylose in der Zusammensetzung 0,9 bis 1,0 beträgt, wobei die Zusammensetzung 0,1 bis 25,0 Gew.-% Dimethylglycin und/oder ein Salz von Dimethylglycin enthält.

2. Zusammensetzung nach Anspruch 1, wobei das Amylopektin aus D-Glucose-Monomeren zusammengesetzt ist, welche α-l,4-glycosidisch miteinander verbunden sind, und wobei etwa alle 10-30 Monomere eine Seitenkette aus D10 Glucose-Monomeren α-l,6-glycosidisch verknüpft ist.

3. Zusammensetzung nach Anspruch 2, wobei die Seitenketten des Amylopektins jeweils etwa 5-60 Monomere, bevorzugt jeweils etwa 10-30 Monomere, stärker bevorzugt jeweils etwa 10-20 Monomere, stärker bevorzugt jeweils etwa 10-15 Monomere umfassen.

4. Zusammensetzung nach einem der vorangehenden Ansprüche, wobei das Amylopektin eine molare Masse von mindestens 100.000 g/mol aufweist.

5. Zusammensetzung nach einem der vorangehenden Ansprüche, wobei die Zusammensetzung weiterhin enthält
c) mindestens einen weiteren Wirkstoff, ausgewählt aus Amla-Extrakt, Amylodextrin, β-Carotin, Biotin, Carnitin, Curcumin, Echinacea, Ectoin, Eisen, Folsäure, Granatapfel -Extrakt, Grüntee-Extrakt, Hirseextrakt, Hirseöl, Kürbiskernöl, Kreatin, L-Cystein, L-Lysin, Niacin, Niacinamid, Pantolacton, Panthothensäure, Piperin, Procyanidin, Rosmarin -Extrakt, Sägepalme-Extrakt, Schachtelhalm-Extrakt, Selen, Silicium, Taurin, Tocopherylacetat, Tomatenextrakt, Ubichinon-10, Vitamin B1, Vitamin B2, Vitamin B6, Vitamin B 12, Vitamin C, Vitamin E, Zink und Kombinationen hiervon.

6. Zusammensetzung nach einem der vorangehenden Ansprüche, wobei die Zusammensetzung ein Nahrungsergänzungsmittel zur Förderung des Stoffwechsels der Haare und/oder der Kopfhaut und/oder zur Förderung des Haarwachstums und/oder zur Behandlung und/oder Vorbeugung von Haarausfall ist.

7. Zusammensetzung nach einem der Ansprüche 1 bis 6 zur Verwendung bei der Behandlung und/oder Vorbeugung von Haarausfall.

8. Zusammensetzung zur Verwendung bei der Behandlung und/oder Vorbeugung von Haarausfall nach Anspruch 7, wobei der Haarausfall ausgewählt ist aus Alopecia areata (kreisrundem Haarausfall), Alopecia androgenetica (erblich bedingter Haarausfall) bei Frauen und Männern, diffuser Alopezie (diffusem Haarausfall), altersbedingtem Haarausfall (senescent Alopecia) und durch Chemotherapie bedingtem Haarausfall.

9. Kosmetische Verwendung der Zusammensetzung nach einem der Ansprüche 1-6.

10. Kosmetische Verwendung nach Anspruch 9 zur Verbesserung des Zustands der Haare und/oder der Kopfhaut und/oder zur Förderung des Stoffwechsels der Haare und/oder der Kopfhaut und/oder zur Förderung des Haarwachstums.

## Claims

1. Oral composition containing
a) dimethylglycine and/or a salt of dimethylglycine, and
b) amylopectin, wherein the weight ratio of the amount of amylopectin in the composition to the total amount of amylopectin and amylose in the composition is 0.9 to 1.0, and wherein the composition contains 0.1 to 25.0 wt.% dimethylglycine and/or a salt of dimethylglycine.

2. Composition according to claim 1, wherein the amylopectin is composed of D-glucose monomers which are joined together by α-1,4-glycosidic bonds, and wherein approximately every 10 to 30 monomers a side chain of D10 glucose monomers is linked by α-1,6-glycosidic bonds.

3. Composition according to claim 2, wherein the side chains of the amylopectin each comprise about 5-60 monomers, preferably about 10-30 monomers, more preferably in each case about 10-20 monomers, more preferably about 10-15 monomers.

4. Composition according to any one of the preceding claims, wherein the amylopectin has a molar mass of at least 100,000 g/mol.

5. Composition according to any one of the preceding claims, wherein the composition further comprises:
c) at least one additional active ingredient selected from amla extract, amylodextrin, β-carotene, biotin, carnitine, curcumin, echinacea, ectoin, iron, folic acid, pomegranate extract, green tea extract, millet extract, millet oil, pumpkin seed oil, creatine, I-cysteine, I-lysine, niacin, niacinamide, pantolactone, pantothenic acid, piperine, procyanidin, rosemary extract, sage palm extract, horsetail extract, selenium, silicon, taurine, tocopheryl acetate, tomato extract, ubiquinone-10, vitamin B1, vitamin B2, vitamin B6, vitamin B12, vitamin C, vitamin E, zinc, and combinations thereof.

6. Composition according to any of the preceding claims, wherein the composition is a dietary supplement to promote hair and/or scalp metabolism and/or promote hair growth and/or treat and/or prevent alopecia.

7. Composition according to any of claims 1 to 6 for use in the treatment and/or prevention of alopecia.

8. Composition for use in the treatment and/or prevention of alopecia according to claim 7, wherein the alopecia is selected from alopecia areata (patchy hair loss), androgenetic alopecia (hereditary hair loss) in women and men, diffuse alopecia (diffuse hair loss), age-related alopecia (senescent alopecia), and alopecia caused by chemotherapy.

9. Cosmetic use of the composition according to any one of claims 1 to 6.

10. Cosmetic use according to claim 9 for improving the condition of the hair and/or scalp and/or for promoting the metabolism of the hair and/or scalp and/or for promoting hair growth.

## Revendications

1. Composition orale, contenant
a) de la diméthylglycine et/ou un sel de diméthylglycine, et
b) de l'amylopectine, le rapport pondéral de la quantité d'amylopectine dans la composition sur la quantité totale d'amylopectine et d'amylose dans la composition étant de 0,9 à 1,0, la composition contenant 0,1 à 25,0 % en poids de diméthylglycine et/ou d'un sel de diméthylglycine.

2. Composition selon la revendication 1,
dans laquelle l'amylopectine est composée de monomères de D-glucose qui sont liés entre eux par des liaisons glycosidiques α-1,4, et
environ tous les 10 à 30 monomères, une chaîne latérale de monomères de D10 glucose est liée par des liaisons glycosidiques α-1,6.

3. Composition selon la revendication 2,
dans laquelle les chaînes latérales de l'amylopectine comprennent chacune environ 5 à 60 monomères, de préférence environ 10 à 30 monomères, de préférence encore environ 10 à 20 monomères, et de manière particulièrement préférée environ 10 à 15 monomères.

4. Composition selon l'une des revendications précédentes,
dans laquelle l'amylopectine a une masse molaire d'au moins 100 000 g/mol.

5. Composition selon l'une des revendications précédentes,
la composition contenant en outre
c) au moins un autre ingrédient actif choisi parmi l'extrait d'amla, l'amylodextrine, le bêta-carotène, la biotine, la carnitine, la curcumine, l'échinacée, l'éctoïne, le fer, l'acide folique, l'extrait de grenade, l'extrait de thé vert, l'extrait de millet, l'huile de millet, l'huile de pépins de courge, la créatine, la L-cystéine, la L-lysine, la niacine, le niacinamide, le pantolactone, l'acide pantothénique, la pipérine, la procyanidine, l'extrait de romarin, l'extrait de palmier scie, l'extrait de prêle, le sélénium, le silicium, la taurine, l'acétate de tocophéryle, l'extrait de tomate, l'ubiquinone 10, la vitamine B1, la vitamine B2, la vitamine B6, la vitamine B 12, la vitamine C, la vitamine E, le zinc et les combinaisons de ceux-ci.

6. Composition selon l'une des revendications précédentes,
la composition étant un complément alimentaire destiné à favoriser le métabolisme des cheveux et/ou du cuir chevelu et/ou à favoriser la croissance des cheveux et/ou à traiter et/ou prévenir la chute des cheveux.

7. Composition selon l'une des revendications 1 à 6,
destinée à être utilisée dans le traitement et/ou la prévention de la chute des cheveux.

8. Composition destinée à être utilisée dans le traitement et/ou la prévention de la chute des cheveux selon la revendication 7,
la chute des cheveux étant choisie parmi l'alopécie areata (chute de cheveux circulaire), l'alopécie androgénétique (chute de cheveux héréditaire) chez les femmes et les hommes, l'alopécie diffuse (chute de cheveux diffuse), la chute de cheveux liée à l'âge (alopécie sénescente) et la chute de cheveux liée à la chimiothérapie.

9. Utilisation cosmétique de la composition selon l'une des revendications 1 à 6.

10. Utilisation cosmétique selon la revendication 9 pour améliorer l'état des cheveux et/ou du cuir chevelu et/ou pour favoriser le métabolisme des cheveux et/ou du cuir chevelu et/ou pour favoriser la croissance des cheveux.
